# EUROPÄISCHE PATENTSCHRIFT

(11) **EP 2 638 010 B1**
(45) Veröffentlichungstag und Bekanntmachung des Hinweises auf die Patenterteilung: **31.12.2014**
(21) Anmeldenummer: 11782114.0
(22) Anmeldetag: 08.11.2011
(51) Int. Cl.: C07D 277/28, C07D 213/14, C07D 213/38, C07C 209/08

(54) **VERFAHREN ZUR HERSTELLUNG VON 2,2-DIFLUORETHYLAMIN-DERIVATEN AUSGEHEND VON N-(2,2-DIFLUORETHYL)PROP-2-EN-1-AMIN**
METHOD FOR PREPARING 2,2-DIFLUOROETHYLAMINE DERIVATIVES STARTING FROM N-(2,2-DIFLUOROETHYL)PROP-2-EN-1-AMINE
PROCÉDÉ DE PRODUCTION DE DÉRIVÉS DE 2,2-DIFLUORO-ÉTHYLAMINE À PARTIR DE N-(2,2-DIFLUORO-ÉTHYL)PROP-2-ÈN-1-AMINE

(30) Priorität: 12.11.2010 US 413008 P; 12.11.2010 EP 10191066
(43) Veröffentlichungstag der Anmeldung: 18.09.2013
(73) Patentinhaber: Bayer Intellectual Property GmbH, 40789 Monheim am Rhein (DE)
(72) Erfinder: LUI, Norbert, 51519 Odenthal (DE); HEINRICH, Jens-Dietmar, 51381 Leverkusen (DE)
(74) Vertreter: BIP Patents
(86) Internationale Anmeldenummer: PCT/EP2011/069625
(87) Internationale Veröffentlichungsnummer: WO 2012/062740

(56) Entgegenhaltungen:
- WO-A1-2007/115644
- WO-A1-2009/036900
- WO-A1-2009/036901

## Beschreibung

Die vorliegende Erfindung betrifft ein Verfahren zur Herstellung bestimmter 2,2-Difluorethylamin-Derivate ausgehend von N-(2,2-Difluorethyl)prop-2-en-1-amin.

2,2-Difluorethylamin-Derivate sind nützliche Zwischenstufen bei der Herstellung agrochemischer Wirkstoffe (siehe z.B. WO 2007/115644). Es sind verschiedene Verfahren zur Herstellung von 2,2-Difluorethylamin-Derivaten, z.B. durch Amidhydrierung oder durch Reduktion mit Wasserstoff, bekannt.

WO 2009/036900 beschreibt beispielsweise ein Verfahren zur Herstellung von 2,2-Difluorethylamin-Derivaten durch Amidhydrierung von N-[(6-Chlorpyridin-3-yl)methyl]-2,2-difluoracetamide (siehe Schema 1), das jedoch durch den Einsatz komplexer Hydride wie Natriumborhydrid nachteilig ist, da Hydride sehr teuer und nur sicherheitstechnisch aufwendig zu verwenden sind.

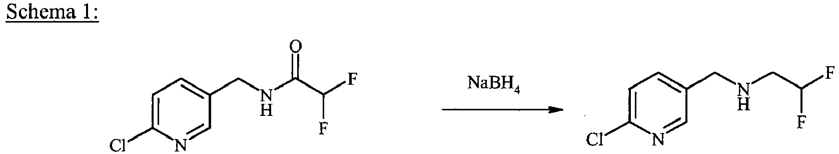

WO 2009/036901 beschreibt die Reduktion von N-(6-Chlorpyridin-3-yl)methylen-2,2-difluorethanamin durch Wasserstoff (siehe Schema 2), während WO 2010/105747 die Reduktion von 1-(6-Chloropyridin-3-yl)-N-[(1E)-2,2-difluoroethylidene]methanamin durch Wasserstoff beschreibt. Nachteilig bei diesen Verfahren ist der Einsatz von Wasserstoff, weil auch hier die Verwendung von Wasserstoff sicherheitstechnisch sehr aufwendig ist.

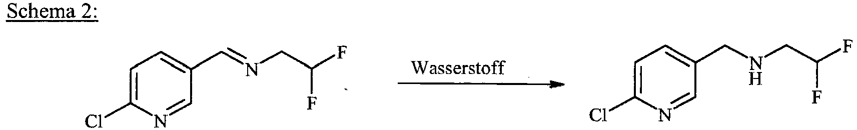

Die Druckschrift WO2007/115644, die sich mit der Herstellung von insektizid wirksamen 4-Aminobut-2-enolidverbindungen befasst, beschreibt die Herstellung von Verbindungen der allgemeinen Formel A-CH₂-NH-R¹, in der A für spezielle Heterocyclen und R¹ für Halogenalkyl steht, durch Alkylierung des Stickstoffs (Schema 3).

Konkret beschreibt WO2007/115644 die Herstellung von *N*-[(6-Chlorpyridin-3-yl)methyl]-2,2-difluorethan-1-amin (Verbindung (3)), das ausgehend von 2-Chlor-5-chlormethyl-pyridin (Verbindung (2)) und 2,2-Difluorethan-1-amin (Verbindung (1)) in Gegenwart von Triethylamin synthetisiert wird (siehe Schema 4). Die Verbindungen (1), (2) und Triethylamin werden dabei in äquimolaren Mengen eingesetzt. Das gewünschte Produkt wird in einer Ausbeute von 53 % erhalten.

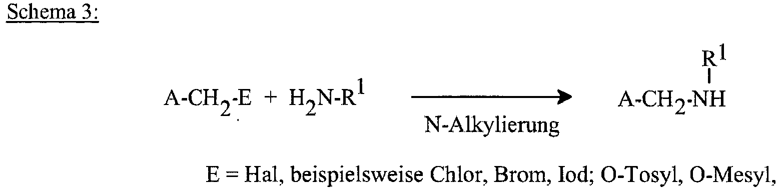

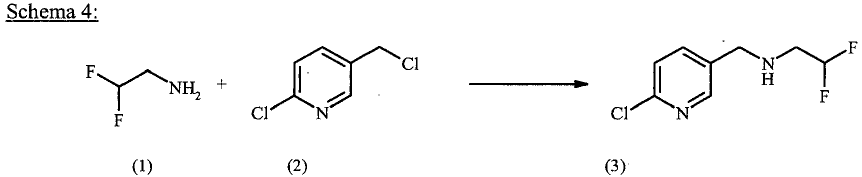

WO 2007/115644 beschreibt ferner, dass die Verbindungen N-[(6-Chlorpyridin-3-yl)methyl]-3-fluor-propan-1-amin, und *N*-[(6-Chlorpyridin-3-yl)methyl]-2-chlor-2-fluorethan-l-amin in gleicher Weise hergestellt wurden.

Das in WO 2007/115644 beschriebene Verfahren zur Herstellung von Verbindungen der Formel A-CH₂-NH-R¹, in der A für spezielle Heterocyclen und R¹ für Halogenalkyl steht, ist nachteilig, da es während der Umsetzung zu Mehrfachalkylierungen des Stickstoffs kommen kann. Dies führt zu einem Ausbeuteverlust, was auch an der Ausbeute des konkret genannten Beispiels zu erkennen ist. Die Ausbeute betrug lediglich 53 %. Diese Mehrfachalkylierungen können nur durch den Einsatz eines großen Überschusses an Amin reduziert werden. Abgesehen davon, dass Amine oft sehr kostenintensiv sind, ist das Verfahren auch deshalb unwirtschaftlich, da das im Überschuss zugegebene und nicht umgesetzte Amin entweder entsorgt oder aufwändig zurückgewonnen werden muss.

Wegen der Bedeutung von 2,2-Difluorethylamin-Derivaten als Bausteine zur Synthese agrochemischer Wirkstoffe ist es jedoch notwendig ein Verfahren zu finden, das großtechnisch und kostengünstig eingesetzt werden kann. Auch ist es erstrebenswert die speziellen 2,2-Difluorethylamin-Derivate mit hoher Ausbeute und hoher Reinheit zu erhalten, so dass die Zielverbindung vorzugsweise keiner weiteren, möglicherweise komplexen, Aufreinigung unterzogen werden muss.

Es wurde nun ein einfaches und deshalb kostengünstiges Verfahren zur Herstellung von 2,2-Difluorethylamin-Derivaten der Formel (IV) gefunden, mit dem die oben genannten Nachteile vermieden werden.

Gegenstand der Erfindung ist somit ein Verfahren zur Herstellung von 2,2-Difluorethylamin-Derivaten der Formel (IV) 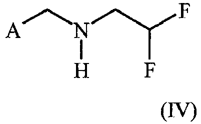 das die folgenden Schritte (i) und (ii) umfasst:
Schritt (i) - Alkylierung: Umsetzung von N-(2,2-Difluorethyl)prop-2-en-1-amin der Formel (I) 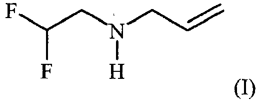 mit einer Verbindung der Formel (II)

   A-CH₂-E (II)

   zu einer Verbindung der Formel (III) 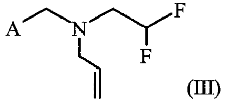
gegebenenfalls in Gegenwart einer anorganischen oder organischen Base,
wobei in den Formeln (II), (III) und (IV)
   - A: für Pyrid-2-yl, Pyrid-4-yl oder Pyrid-3-yl steht, die gegebenenfalls in 6-Position durch Fluor, Chlor, Brom, Methyl, Trifluormethyl oder Trifluormethoxy substituiert sein können, oder für 1,3-Thiazol-5-yl steht, das in 2-Position durch Chlor oder Methyl substituiert sein kann, oder für Pyrid-3-yl der folgenden Formel 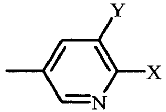 steht, in der
   - X: für Halogen, C₁-C₁₂-Alkyl oder C₁-C₁₂-Halogenalkyl steht und
   - Y: für Halogen, C₁-C₁₂-Alkyl, C₁-C₁₂-Halogenalkyl, C₁-C₁₂-Halogenalkoxy, Azido oder Cyan steht, bevorzugt steht A für 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 2-Chlor-1,3-thiazol-5-yl oder 2-Methyl-1,3-thiazol-5-yl, 5,6-Difluor-pyrid-3-yl, 5-Chlor-6-fluor-pyrid-3-yl, 5-Brom-6-fluor-pyrid-3-yl, 5-Fluor-6-chlor-pyrid-3-yl, 5,6-dichlor-pyrid-3-yl, 5-Brom-6-chlor-pyrid-3-yl, 5-Fluor-6-brom-pyrid-3-yl, 5-Chlor-6-brom-pyrid-3-yl, 5-Methyl-6-fluor-pyrid-3-yl, 5-Methyl-6-chlor-pyrid-3-yl, 5-Methyl-6-brom-pyrid-3-yl, 5-Difluormethyl-6-fluor-pyrid-3-yl oder 5-Difluormethyl-6-chlor-pyrid-3-yl. Besonders bevorzugt steht A für 6-Fluor-pyrid-3-yl, 6-Chlor-pyrid-3-yl, 6-Brompyrid-3-yl, 2-Chlor-1,3-thiazol-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl oder 5-Difluormethyl-6-chlor-pyrid-3-yl. Ganz besonders bevorzugt steht A für 6-Chlor-pyrid-3-yl, 6-Brom-pyrid-3-yl, 2-Chlor-1,3-thiazol-5-yl, 5-Fluor-6-chlor-pyrid-3-yl oder 5-Fluor-6-brom-pyrid-3-yl,
   und in Formel (II)
   - E: für eine Abgangsgruppe steht, insbesondere für Halogen steht (z.B. Chlor, Brom oder Jod) oder für eine aktivierte Hydroxyverbindung steht (z.B. Mesylat, Tosylat oder SO₂CH₃),
   bevorzugt steht E für Chlor, Brom oder Mesylat,
   und
Schritt (ii): Entfernung der Allylgruppe (Deallylierung) aus der in Schritt (i) erhaltenen Verbindung der Formel (III), wodurch ein Difluorethylamin-Derivat der Formel (IV) oder einem Salz davon erhalten wird, vorzugsweise in Gegenwart eines Katalysators und gegebenenfalls in Gegenwart eines Nucleophils.

Das erfindungsgemäße Verfahren kann durch das folgende Schema 5 veranschaulicht werden:

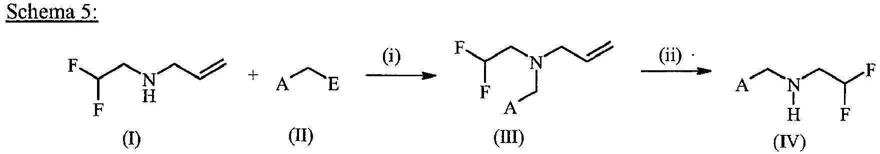

Das gewünschte 2,2-Difluorethylamin-Derivat der Formel (IV) wird mit dem erfindungsgemäßen Verfahren mit guten Ausbeuten, kurzer Reaktionszeit und in hoher Reinheit erhalten, weshalb eine umfangreiche Aufarbeitung des unmittelbaren Reaktionsprodukts im Allgemeinen nicht erforderlich ist, insbesondere weil die Reaktion nur eine einfache Alkylierung zulässt und somit die Bildung von mehrfachalkylierten Produkten verhindert.

Das erfindungsgemäße Verfahren hat gegenüber dem in WO2007/115644 beschriebenen Verfahren den Vorteil, dass bessere Ausbeuten erzielt werden und es deshalb ökologisch und wirtschaftlich sinnvoll ist.

Gegenstand der Erfindung ist gleichfalls das Verfahren des Schritts (i) zur Herstellung einer Verbindung der Formel (III) 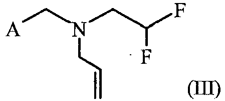 umfassend die Umsetzung von N-(2,2-Difluorethyl)prop-2-en-1-amin der Formel (I) mit einer Verbindung der Formel (II) das die für Schritt (i) beschriebenen Verfahrensschritte, Reaktionsbedingungen und Reaktionspartner beinhaltet.

Gegenstand der Erfindung ist gleichfalls die Verbindung der Formel (III) 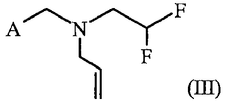 worin
- A: für Pyrid-2-yl, Pyrid-4-yl oder Pyrid-3-yl steht, die gegebenenfalls in 6-Position durch Fluor, Chlor, Brom, Methyl, Trifluormethyl oder Trifluormethoxy substituiert sein können, oder für 1,3-Thiazol-5-yl steht, das in 2-Position durch Chlor oder Methyl substituiert sein kann, oder für Pyrid-3-yl der folgenden Formel 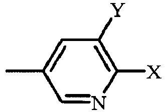 steht, in der
- X: für Halogen, C₁-C₁₂-Alkyl oder C₁-C₁₂-Halogenalkyl steht und
- Y: für Halogen, C₁-C₁₂-Alkyl, C₁-C₁₂-Halogenalkyl, C₁-C₁₂-Halogenalkoxy, Azido oder Cyan steht, bevorzugt steht A für 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 2-Chlor-1,3-thiazol-5-yl oder 2-Methyl-1,3-thiazol-5-yl, 5,6-Difluor-pyrid-3-yl, 5-Chlor-6-fluor-pyrid-3-yl, 5-Brom-6-fluor-pyrid-3-yl, 5-Fluor-6-chlor-pyrid-3-yl, 5,6-dichlor-pyrid-3-yl, 5-Brom-6-chlor-pyrid-3-yl, 5-Fluor-6-brom-pyrid-3-yl, 5-Chlor-6-brom-pyrid-3-yl, 5-Methyl-6-fluor-pyrid-3-yl, 5-Methyl-6-chlor-pyrid-3-yl, 5-Methyl-6-brom-pyrid-3-yl, 5-Difluormethyl-6-fluor-pyrid-3-yl oder 5-Difluormethyl-6-chlor-pyrid-3-yl. Besonders bevorzugt steht A für 6-Fluor-pyrid-3-yl, 6-Chlor-pyrid-3-yl, 6-Brompyrid-3-yl, 2-Chlor-1,3-thiazol-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl oder 5-Difluormethyl-6-chlor-pyrid-3-yl. Ganz besonders bevorzugt steht A für 6-Chlor-pyrid-3-yl, 6-Brom-pyrid-3-yl, 2-Chlor-1,3-thiazol-5-yl, 5-Fluor-6-chlor-pyrid-3-yl oder 5-Fluor-6-brom-pyrid-3-yl.

Gegenstand der Erfindung ist weiterhin die Verwendung der Verbindung der Formel (III) 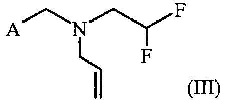 zur Herstellung von 2,2-Difluorethylamin, die die für Schritt (ii) beschriebenen Verfahrensschritte, Reaktionsbedingungen und Reaktionspartner umfasst.

Im Rahmen der vorliegenden Erfindung wird unter einem Derivat ein von dem bezeichneten organischen Grundgerüst (Baustein) abgeleiteter Stoff ähnlicher Struktur verstanden, d.h. unter einem 2,2-Difluorethylamin-Derivat wird insbesondere eine Verbindung verstanden, welche einen 2,2-Difluorethylamin-Baustein umfasst.

Sofern nichts anderes angegeben, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Sofern nichts anderes angegeben, wird unter dem Begriff "Aryl" ein aromatischer Rest mit 6 bis 14 Kohlenstoffatomen, vorzugsweise Phenyl, verstanden.

Im Rahmen der vorliegenden Erfindung werden unter durch Halogen substituierte Reste, beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Jod.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Gegebenenfalls substituierte Reste können einfach oder mehrfach substituiert sein, wobei bei einer Mehrfachsubstitution die Substituenten gleich oder verschieden sein können.

Verbindungen der Formel (I) können wie in der Europäischen Patentanmeldung Nr. 10191059.4 für Schritt (i) beschrieben, hergestellt werden. Insofern wird auf diese Anmeldung vollumfänglich Bezug genommen.

Verbindungen der Formel (I) werden durch die Umsetzung von 2,2-Difluor-1-halogenethan mit folgender Formel CHF₂-CH₂Hal, worin Hal für Chlor, Brom oder Jod steht, mit Prop-2-en-1-amin, vorzugsweise in Gegenwart einer organischen oder anorganischen Base, hergestellt. Die Umsetzung wird gewöhnlich in Substanz durchgeführt, und Prop-2-en-1-amin dient gleichzeitig als Säurefänger. Mehrere in der Europäischen Patentanmeldung beschriebene Herstellungsverfahren sind weiter unten beschrieben.

Die Verbindungen der Formel (II) sind teilweise bekannt, und sogar käuflich erhältlich, oder können nach bekannten Methoden hergestellt werden (z.B. die Verbindung 2-Chlor-5-chlormethyl-1,3-thiazol gemäß DE-A-3 631 538, EP-A-446 913, EP-A-780 384, EP-A-775 700, EP-A-794 180, WO 97/ 0010226, die Verbindung 6-Chlor-3-chlormethyl-pyridin gemäß DE-A1-3 630 046, EP-A2-373 464, EP-A2-393 453, EP-A1-569 947, die Verbindung 6-Chlor-3-brommethyl-pyridin gemäß Cabanal-Duvillard, I. et al. (Heterocycl. Commun. 5, 257-262 (1999)), die Verbindungen 6-Brom-3-chlormethyl-pyridin, 6-Brom-3-hydroxymethyl-pyridin gemäß US 5,420,270 B, die Verbindung 6-Fluor-3-chlormethyl-pyridin gemäß Pesti, J. A. et al. (J. Org. Chem. 65, 7718-7722 (2000)), die Verbindung 6-Methyl-3-chlormethyl-pyridin gemäß EP-A2-302389 oder nach Van der Eycken, E. et al. (J. Chem. Soc., Perkin Trans (2) 5, 928-937 (2002)), die Verbindung 6-Trifluormethyl-3-chlormethyl-pyridin gemäß WO 2004/082616, oder die Verbindung 2-Chlor-5-chlormethyl-pyrazin gemäß JP 1993-239034 A2).

Allgemeine Wege zur Herstellung von Verbindungen der Formel (II) sind im folgenden Schema 6 wiedergegeben.

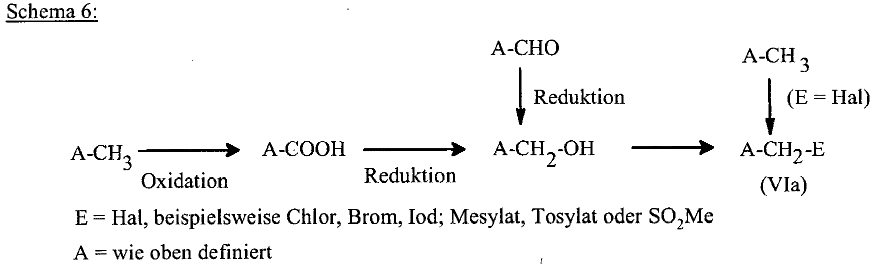

Beispielsweise können die heterocyclischen Carbonsäuren (A-COOH) nach literaturbekannten Methoden in die entsprechenden heterocyclischen Hydroxymethylverbindungen (A-CH₂-OH) überführt werden, die anschließend nach literaturbekannten Methoden zu aktivierten heterocyclischen Hydroxymethylverbindungen (A-CH₂-E, E = Tosylat, Mesylat) oder heterocyclischen Halogenmethylverbindungen (A-CH₂-E, E = Hal) umgesetzt werden. Letztere können auch aus entsprechenden Methylgruppe-haltigen Heterocyclen (A-CH₃) unter Verwendung von geeigneten literaturbekannten Halogenierungsmitteln erhalten werden.

Die Umsetzung von N-(2,2-Difluorethyl)prop-2-en-1-amin der Formel (I) mit A-CH₂-E der Formel (II) in Schritt (i) kann in Substanz, d.h. ohne Hinzufügen eines Lösungsmittels oder in Gegenwart eines Lösungsmittels durchgeführt werden.

Im Falle dass in Schritt (i) ein Lösungsmittel zum Reaktionsgemisch hinzugefügt wird, wird es vorzugsweise in einer solchen Menge eingesetzt, dass das Reaktionsgemisch während des ganzen Verfahrens gut rührbar bleibt. Vorteilhafterweise wird, bezogen auf das Volumen des eingesetzten 2,2-Difluor-1-halogenethan, die 1- bis 30-fache Lösungsmittelmenge, bevorzugt die 2- bis 20-fache Lösungsmittelmenge, besonders bevorzugt die 2- bis 15-fache Lösungsmittelmenge verwendet. Unter Lösungsmittel werden erfindungsgemäß auch Gemische reiner Lösungsmittel verstanden. Geeignete Lösungsmittel sind alle unter den Reaktionsbedingungen inerten organischen Lösungsmittel. Erfindungsgemäß geeignete Lösungsmittel sind insbesondere Ether (z.B. Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n*-butylether, Diisobutylether, Diisoamylether, Ethylenglycoldimethylether, Isopropylethylether, Diethylenglykoldimethylether, Triethylengylkoldimethylether, Tetrahydrofuran, 2-Methyltetrahydrofuran, Dioxan, und Polyether des Ethylenoxids und/oder Propylenoxids); Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Oktan, Benzol, Toluol, Xylol); halogenierte Kohlenwasserstoffe, wie Dichlormethan, Chloroform, Tetrachlorkohlenstoff, Dichlorethan oder Trichlorethan; halogenierte Aromate (z.B. Chlorbenzol, Dichlorbenzol); Amide (z.B. Hexamethylphosphorsäuretriamid, Formamid, N,N-Dimethyl-acetamid, *N*-Methyl-formamid, *N,N-*Dimethyl-formamid, *N,N-*Dipropyl-formamid, *N,N*-Dibutyl-formamid, *N*-Methyl-pyrrolidin, *N*-Methylcaprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N*'-1,4-Diformyl-piperazin); Nitrile (z.B. Acetonitril, Propionitril, n- oder iso-Butyronitril oder Benzonitril); Alkohole wie z.B. Methanol, Ethanol, n-Propanol, iso-Propanol, n-Butanol, sek.-Butanol, tert.-Butanol; Ketone (z.B. Aceton); oder Gemische davon.

Bevorzugte Lösungsmittel in Schritt (i) sind aromatische und/oder aliphatische Kohlenwasserstoffe, insbesondere Toluol, N,N-Dimethylacetamid und *N*-Methyl-pyrrolidon.

Es ist erfindungsgemäß bevorzugt Schritt (i) in Substanz, d.h. ohne Lösungsmittel durchzuführen. Hierdurch kann das Verfahren noch kostengünstiger durchgeführt werden, weil die Lösungsmittel weder gekauft, noch nach Reaktion entsorgt werden müssen.

Als Abgangsgruppe E sind Gruppen geeignet, die bei den vorherrschenden Reaktionsbedingungen eine ausreichende Nucleofugizität aufweisen. Insbesondere sind Halogene, wie Chlor, Brom, oder Jod, oder Mesylat, Tosylat oder SO₂CH₃ geeignete Abgangsgruppen. Bevorzugte Abgangsgruppen E sind Chlor, Brom und Mesylat.

Die Reaktion in Schritt (i) erfolgt vorteilhafterweise in Gegenwart einer geeigneten Base, wie beispielsweise einer anorganischen oder organischen Base.

In Schritt (i) kann insbesondere eine oder mehrere der folgenden anorganischen Basen verwendet werden: Hydride, Hydroxide, Amide, Alkoholate, Acetate, Fluoride, Phosphate, Carbonate und Hydrogencarbonate von Alkali- oder Erdalkalimetallen. Bevorzugte Basen sind Natriumamid, Natriumhydrid, Lithiumdiisopropylamid, Natriummethanolat, Kalium-tert-butanolat, Natriumhydroxid, Kaliumhydroxid, Natriumacetat, Natriumphosphat, Kaliumphosphat, Kaliumfluorid, Caesiumfluorid, Natriumcarbonat, Kaliumcarbonat, Kaliumhydrogencarbonat, Natriumhydrogencarbonat und Caesiumcarbonat. Die anorganische Base wird gegebenenfalls als wässrige Lösung in einer Konzentration im Bereich von etwa 10 und 40 Gew.-% eingesetzt.

Gleichfalls kann in Schritt (i) insbesondere eine oder mehrere der folgenden organischen Basen verwendet werden: Tertiäre Amine, substituierte oder unsubstituierte Pyridine und substituiertes oder unsubstituiertes Triethylamin, Trimethylamin, N,N-Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylethylendiamin, N,N-Dimethyl-1,4-diazacyclohexan, N,N-Di-ethyl-1,4-diazacyclohexan, 1,8-Bis-(Dimethyl-amino)naphthalin, Diazabicyclooctan (DABCO), Diazabicyclononan (DBN), Diazabicycloundecan (DBU), Butylimidazol und Methylimidazol.

Das molare Verhältnis von Base zum eingesetzten N-(2,2-Difluorethyl)prop-2-en-1-amin der Formel (I) liegt im Bereich von etwa 0,1 bis etwa 10, bevorzugt im Bereich von etwa 0,5 bis etwa 4, besonders bevorzugt im Bereich von etwa 1 bis etwa 3. Der Einsatz größerer Mengen an Base ist möglich, jedoch aus wirtschaftlichen Gründen nachteilig. Die Base kann gleichzeitig auch Lösungsmittel sein.

Das molare Verhältnis der Verbindung der Formel (II) zum eingesetzten N-(2,2-Difluorethyl)prop-2-en-1-amin der Formel (I) liegt normalerweise im Bereich von etwa 0,5 bis etwa 3, bevorzugt im Bereich von etwa 0,7 bis etwa 2, besonders bevorzugt im Bereich von 0,8 bis etwa 1,5. Der Einsatz größerer Mengen an Verbindung der Formel (II), das als Alkylierungsmittel dient, ist grundsätzlich möglich, jedoch wirtschaftlich nachteilig.

Die Verbindung der Formel (II) kann zu dem N-(2,2-Difluorethyl)prop-2-en-1-amin der Formel (I) zudosiert (zugegeben) werden oder auch umgekehrt. Die Verbindung der Formel (II) und N-(2,2-Difluorethyl)prop-2-en-1-amin der Formel (I) können auch gleichzeitig zudosiert werden.

Obwohl Schritt (i) des erfindungsgemäßen Verfahrens im Allgemeinen ohne Zusatz eines Katalysators durchgeführt wird, können im Schritt (i) auch Katalysatoren, die die Umsetzung der Verbindung der Formel (II) mit N-(2,2-Difluorethyl)prop-2-en-1-amin der Formel (I) beschleunigen, verwendet werden. Mischungen geeigneter Katalysatoren sind auch denkbar.

Geeignet sind beispielsweise Alkalibromide und -jodide (z.B. Natriumjodid, Kaliumjodid, Kaliumbromid); Ammoniumbromid und Ammoniumjodid; Tetraalkylammoniumbromide und -jodide, (z.B. Tetraethylammoniumjodid); bestimmte Phosphoniumhalogenide, wie Tetraalkyl- oder Tetraarylphosphoniumhalogenide (z.B. Hexadecyl-tri-butyl-phosphoniumbromid, Stearyltributylphosphoniumbromid, Tetrabutylphosphoniumbromid, Tetraoctylphosphoniumbromid, Tetraphenylphosphoniumchlorid und Tetraphenylphosphoniumbromid), Tetrakis(dimethylamino)phosphoniumbromid, Tetrakis(diethylamino)phosphoniumbromid, Tetrakis(dipropylamino)phosphoniumchlorid und -bromid; sowie Bis(dimethylamino)[(1,3-dimethylimidazolidin-2-yliden)amino]methyliumbromid.

Von den vorgenannten Katalysatoren sind Natriumjodid, Kaliumjodid, Kaliumbromid, Tetrabutylammoniumbromid oder Tetraphenylphosponiumbromid besonders geeignet, die Umsetzung des Schritts (i) zu beschleunigen. Natrium- und Kaliumjodid sind besonders hervorzuheben.

Der Katalysator kann auch *in-situ* erzeugt werden, beispielsweise durch eine Reaktion von HBr oder HJ mit Ammoniak. Weiterhin kann der Katalysator auch *in-situ* durch Zugabe von sehr reaktiven Alkylbromiden oder -jodiden (z.B. Methyl- oder Ethylbromid oder -jodid) erzeugt werden.

Falls im Schritt (i) ein Katalysator anwesend ist, so wird er, bezogen auf die eingesetzte Verbindung der Formel (II), in einer Konzentration von etwa 0,01 bis etwa 25 Gew.-% verwendet. Höhere Konzentrationen sind grundsätzlich möglich. Bevorzugt wird der Katalysator in einer Konzentration von etwa 0,2 bis etwa 25 Gew.-%, besonders bevorzugt von etwa 0,4 bis etwa 20 Gew.-%, ganz besonders bevorzugt von etwa 0,5 bis etwa 15 Gew.-%. verwendet. Der Katalysator kann aber auch bevorzugt in einer Konzentration von etwa 0,05 bis etwa 3 Gew.-%, von etwa 0,1 bis etwa 10 Gew.-% oder von etwa 0,5 bis etwa 10 Gew.-%. eingesetzt werden.

Die Reaktionstemperatur in Schritt (i) kann in Abhängigkeit der verwendeten Ausgangsstoffe variieren. Schritt (i) kann bei Temperaturen im Bereich von etwa -30°C bis etwa 200°C durchgeführt werden. Es ist bevorzugt, dass bei Durchführung des Reaktionsschrittes (i) die Innentemperatur im Bereich von etwa 10°C bis etwa 150°C, besonders bevorzugt im Bereich von etwa 25°C bis etwa 130°C liegt.

Die Reaktionsdauer der Umsetzung in Schritt (i) liegt im Bereich von etwa 0,5 bis etwa 20 Stunden. Eine längere Reaktionsdauer ist möglich, jedoch wirtschaftlich nicht sinnvoll.

Die Aufarbeitung des Reaktionsgemisches aus Schritt (i) erfolgt entweder durch Filtration und anschließender fraktionierter Destillation oder durch Verwässerung der Reaktionsmischung, anschließender Phasentrennung und nachfolgender fraktionierter Destillation.

Die Allylgruppe in der Verbindung der Formel (III) wird dann im Schritt (ii) wieder entfernt (abgespalten). Dieser Vorgang wird Deallylierung genannt.

Methoden zur Spaltung einer allylischen C-N Bindung sind bekannt und beispielsweise in dem Übersichtssartikel von Escoubet, Stephanie; Gastaldi, Stephane; Bertrand, Michele in European Journal of Organic Chemistry (2005), (18), 3855-3873 beschrieben. In Bezug auf die Durchführung des Schritts (ii) wird auf diese Methoden hier vollumfänglich Bezug genommen. Die "Tsuji-Trost-Reaktion" ist gleichfalls eine Deallylierung. Sie ist die Palladium-katalysierte Allylierung von Nucleophilen wie C-aciden Verbindungen, Enolaten, Aminen und Phenolen mit Allylverbindungen wie Allylacetaten oder Allylbromiden.

Die Deallylierung kann durch Isomerisierung der Doppelbindung der Allylgruppe zu einem Enamin erfolgen, welches dann durch Hydrolyse gespalten werden kann (Reaktionsweg (2) in Schema 7) oder die Allylgruppe kann auf ein anionisches Nucleophil (Nu) übertragen und das 2,2-Difluorethylamin freigesetzt werden (Reaktionswege (1) in Schema 7).

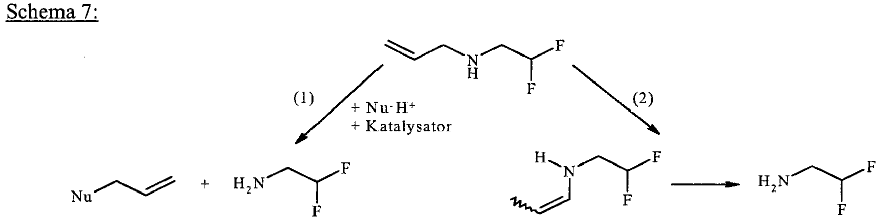

Erfolgt die Deallylierung wie in Schema 7 dargestellt nach Reaktionsweg (2) dann muss in Schritt (ii) eine Säure zur Spaltung des Enamins vorhanden sein. Beispiele solcher Säuren sind Methansulfonsäure, p-Toluolsulfonsäure, Ameisensäure und Essigsäure. Die Reaktionsbedingungen zur Abspaltung der Allylgruppe sind nämlich so zu wählen, dass das gebildete 2,2-Difluorethylamin stabil ist, insbesondere werden keine starken Basen zur Umlagerung verwendet, da es sonst zu Produktverlusten kommt. Starke Basen sind solche Basen bei denen die Gleichgewichtsreaktionen vollständig auf der Seite der OH⁻-Ionen liegen.

In einer bevorzugten Ausführungsform des Schritts (ii) findet die Entfernung der Allylgruppe aus N-(2,2-Difluorethyl)prop-2-en-1-amin in Gegenwart eines geeigneten Katalysators statt. Geeignete Katalysatoren sind heterogene oder homogene Katalysatoren, die ein oder mehrere Metalle der Gruppen 8 - 10 des Periodensystems enthalten. Die entsprechenden Katalysatoren können auch in geträgerter Form, beispielsweise auf Kohlenstoff (Kohle oder Aktivkohle), Aluminiumoxid, Bariumsulfat, Bariumcarbonat, Siliciumdioxid, Zirkondioxid, Calciumcarbonat oder Titandioxid, aufgebracht, verwendet werden. Geeignete Metalle sind insbesondere Edelmetalle (z.B. Ruthenium, Palladium, Platin und Rhodium). Als homogene Katalysatoren eignen sich Palladium(II)chlorid, Palladium(II)acetat, Bis(acetylacetonat)palladium(II), Dichlorobis(triphenylphosphin)palladium(II), Tetrakis(triethylphosphin)palladium, Tetrakis(triphenylphosphin)palladium, und Ruthenium(III)chlorid. Bevorzugt sind Palladium(0)-Katalysatoren, insbesondere Palladium-10%ig auf Kohle. Gleichfalls geeignet sind Palladium(II)chlorid, Palladium(II)acetat, Bis(acetylacetonat)palladium(II), Dichlorobis(triphenylphosphin)palladium(II), Tetrakis(triethylphosphin)palladium, und Tetrakis(triphenylphosphin)palladium. Die Katalysatoren können sowohl in ihrer wasserfeuchten als auch trockenen Form eingesetzt werden.

Findet die Deallylierung des Schritts (ii) in Gegenwart eines Katalysators statt, dann wird der Katalysator, bezogen auf die eingesetzte Verbindung der Formel (IV), in einer Konzentration von etwa 0,001 bis etwa 20 Mol.-% verwendet. Bevorzugt wird der Katalysator in einer Konzentration von etwa 0,01 bis etwa 10 Mol.-% verwendet, besonders bevorzugt von etwa 0,01 bis etwa 5,0 Mol.-%.

Findet die Deallylierung des Schritts (ii) in Gegenwart eines Katalysators statt, dann ist es vorteilhaft, wenn eine Verbindung anwesend ist, die als Nucleophil fungiert. Typische Verbindungen, die als Nucleophile fungieren, und deshalb Nucleophile genannt werden, sind anionische Nucleophile wie Hydroxide, Alkoholate, Thiolate, Carbanionen, Halogenide, Peroxide, Cyanide und Azide. Die anionischen Nucleophile können in protonierter Form eingesetzt werden. Solche protonierten Nucleophile sind z.B. Thiole, Sulfinsäuren, 2-Mercaptöbenzoesäure, Malonsäure und deren Derivate , ß-Dicarbonylverbindungen (z.B. Barbitursäuren, wie N,N'-Dimethylbarbitursäure), und Amine (z.B. Ethanolamin).

Es ist im Allgemeinen vorteilhaft Schritt (ii) in Gegenwart eines Lösungsmittels (Verdünnungsmittel) oder von Lösungsmittelgemischen durchzuführen. Lösungsmittel werden gewöhnlich in einer solchen Menge eingesetzt, dass das Reaktionsgemisch während der Deallylierung gut rührbar bleibt. Als Lösungsmittel zur Durchführung von Schritt (ii) kommen alle unter den Reaktionsbedingungen inerten organischen Lösungsmittel in Frage, wobei die Art des verwendeten Lösungsmittels von der Art der Deallylierung abhängig ist.

Als Beispiele sind zu nennen Alkohole wie Methanol, Ethanol, Isopropanol, Butanol; Ether, wie Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n-*butylether, Diisobutylether, Diisoamylether, Ethylenglycoldimethylether, Isopropylethylether, Tetrahydrofuran, Methyl-Tetrahydrofuran, Dioxan, Dichlordiethylether und Polyether des Ethylenoxids und/oder Propylenoxids; Amine wie Trimethyl-, Triethyl-, Tripropyl-, Tributylamin, *N-*Methylmorpholin, Pyridin, alkylierte Pyridine und Tetramethylendiamin; aliphatische, cycloaliphatische oder aromatische Kohlenwasserstoffe wie Pentan, n-Hexan, n-Heptan, n-Oktan, Nonan und technische Kohlenwasserstoffe welche durch Fluor- und Chloratome substituiert sein können, wie Dichlormethan, Trichlormethan, Tetrachlorkohlenstoff, Fluorbenzol, Chlorbenzol oder Dichlorbenzol; Cyclohexan, Methylcyclohexan, Petrolether, Ligroin, Oktan, Benzol, Toluol, Brombenzol, Nitrobenzol, Xylol; Ester wie Methyl-, Ethyl-, Butyl-, Isobutylacetat, sowie Dimethyl-, Dibutyl-, Ethylencarbonat; Wasser; organische Säuren wie Ameisensäure, Essigsäure, Trifluoressigsäure, Propionsäure und anorganische Säuren wie Schwefelsäure, Salzsäure, Phosphorsäure.

Von den zuvor genannten Lösungsmitteln sind Wasser, Ethanol und Butanol bevorzugt.

Die Aufarbeitung des Reaktionsgemisches nach Schritt (ii) sowie die Aufreinigung des entsprechenden 2,2-Difluorethylamin-Derivats der Formel (IV) können z.B. durch Destillation durchgeführt werden oder über die entsprechenden Salze (z.B. Salze organischer oder anorganischer Säuren (z.B. Hydrochloride oder Acetate). Normalerweise wird das Reaktionsgemisch auf Wasser gegossen und der pH Wert der resultierenden Lösung auf 12 eingestellt. Durch Extraktion mit einem Lösungsmittel wird das 2,2-Difluorethylamin-Derivat der Formel (IV) extrahiert und anschließend vorzugsweise durch Destillation unter Normaldruck oder im Vakuum, isoliert.

Die Aufreinigung eines Salzes eines 2,2-Difluorethylamin-derivats der Formel (IV), beispielsweise Salze organischer oder anorganischer Säuren (z.B. Hydrochloride oder Acetate), erfolgt bevorzugt durch Kristallisation. Wasserlösliche Salze können durch Extraktion der wässrigen Lösungen gereinigt werden, wobei durch die anschließende Umsetzung mit organischen oder anorganischen Basen, bevorzugt NaHCO₃, Na₂CO₃ oder NaOH, das gewünschte 2,2-Difluorethylamin-Derivat der Formel (IV) freigesetzt wird.

Die vorliegende Erfindung wird anhand der nachfolgenden Beispiele näher erläutert, ohne die Erfindung auf diese einzuschränken.
**Herstellung der Ausgangsverbindung der Formel (I) gemäß Europäischer Patentanmeldung Nr.10191059.4**

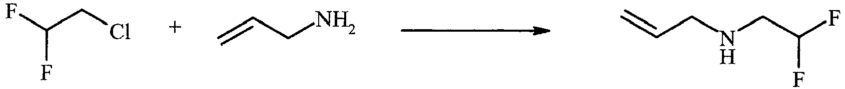

### Variante 1:

382 g (3,67 mol) 2,2-Difluor-1-chlorethan und 70 g (1,2 mol) Prop-2-en-1-amin werden 16 Stunden bei 120°C in einem Autoklaven erhitzt. Das Reaktionsgemisch wird mit 200 g Wasser versetzt und anschließend die Phasen getrennt. Die organische Phase wird bei 55°C destilliert. Man erhält 65 g (entspricht 87,4 % Ausbeute bezogen auf umgesetztes Prop-2-en-1-amin) N-(2,2-Difluorethyl)prop-2-en-1-amin. Unverbrauchtes Prop-2-en-1-amin, das als Hydrochlorid anfällt, kann durch Zugabe von Natronlauge wieder freigesetzt werden.

NMR ¹H (CDCl₃ ) : 5,76 - 6,0 (m, 2H), 5,22 (m, 1H), 3,31 (m, 2H), 2,96 (dt, 2H)

### Variante 2:

382 g (3,67 mol) 2,2-Difluor-1-chlorethan und 70 g Prop-2-en-1-amin (1,2 mol) werden 16 Stunden bei 120°C in einem Autoklaven erhitzt. Das Rohgemisch wird anschließend filtriert und der Rückstand mit 150 g 2,2-Difluor-1-chlorethan gewaschen. Die organische Phase wird zuerst bei Normaldruck und 55°C destilliert. Restmengen an 2,2-Difluor-1-chlorethan werden bei 500 mbar entfernt und der Rückstand wird im Vakuum feindestilliert. Man erhält 56 g (entspricht 76 % Ausbeute) N-(2,2-Difluorethyl)prop-2-en-1-amin. Unverbrauchtes Prop-2-en-1-amin, das als Hydrochlorid anfällt, kann durch Zugabe von Natronlauge wieder freigesetzt werden.

NMR ¹H (CDCl₃ ): 5,76 - 6,0 (m, 2H), 5,22 (m, 1H), 3,31 (m, 2H), 2,96 (dt, 2H)

### Erfindungsgemäßes Beispiel 1 - Schritt (i):

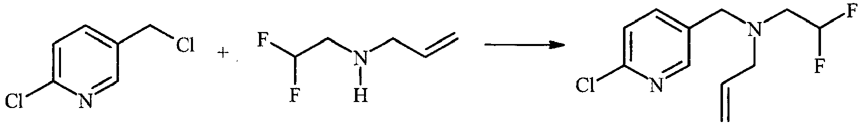

16,46 g (0,135 mol) N-(2,2-Difluorethyl)prop-2-en-1-amin werden in 31,9 g (0,244 mol) N,N-Diisopropylethylamin vorgelegt und bei 70 °C 20 g (0,122 mol) 2-Chlor-5-(chlormethyl)pyridin zudosiert. Die Mischung wird 16 Stunden bei 70°C erhitzt und das überschüssige N,N-Diisopropylethylamin wird anschließend abdestilliert. Der Rückstand wird mit 100 ml Wasser versetzt und zweimal mit 50 ml Dichlormethan extrahiert. Nach Trocknung der vereinigten organischen Phasen über Magnesiumsulfat werden sie über einer Kieselgelschicht filtriert und das Lösungsmittel im Vakuum entfernt. Man erhält 30,3 g (98% Gehalt) N-[(6-Chlorpyridin-3-yl)methyl]-N-(2,2-difluorethyl)prop-2-en-1-amin (entspricht 97,9 % Ausbeute).
NMR ¹H (CDCl₃ ): 8,35 (m, 1H); 7,70 (m, 1H); 7,30 (m, 1H); 5,89 - 5,78 (tt und m, CF₂H und CH); 5,2 (m, 2H); 3,72 (s, 2H); 3,18 (d, 2H), 2,86 (dt, 2H)

### Erfindungsgemäßes Beispiel 2 - Schritt (ii)

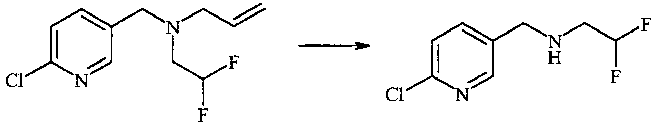

2 g (7,38 mmol) N-[(6-Chlorpyridin-3-yl)methyl]-N-(2,2-difluorethyl)prop-2-en-1-amin werden in 20 ml n-Butanol vorgelegt und mit 100 mg Palladium 10 % auf Kohle (wasserfeucht) versetzt. Anschließend wird 18 h bei Rückfluss erhitzt bis zum vollständigen Umsatz gerührt. Das Reaktionsgemisch wird auf Raumtemperatur abgekühlt und dann über Celite filtriert. Das Lösungsmittel wird im Vakuum entfernt. Man erhält 1,3 g (entspricht 84 % Ausbeute) an N-[(6-Chlorpyridin-3-yl)methyl]-2,2-difluorethanamin.

NMR ¹H (CDCl₃ ): 5,5 - 5,9 (m, 1H), 2,94 - 3,1 (m, 2 H), 1,26 (br m, NH₂)

## Patentansprüche

1. Ein Verfahren zur Herstellung von 2,2-Difluorethylamin-Derivaten der Formel (IV) 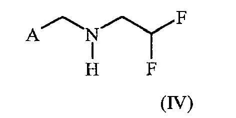 das die folgenden Schritte (i) und (ii) umfasst:
Schritt (i): Umsetzung von N-(2,2-Difluorethyl)prop-2-en-1-amin der Formel (I) 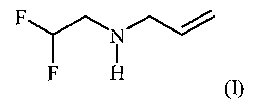 mit einer Verbindung der Formel (II)
A-CH₂-E (II)
zu einer Verbindung der Formel (III) 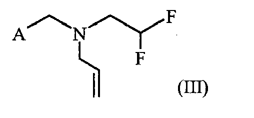 gegebenenfalls in Gegenwart einer anorganischen oder organischen Base,
wobei in den Formeln (II), (III) und (IV)
A für Pyrid-2-yl, Pyrid-4-yl oder Pyrid-3-yl steht, die gegebenenfalls in 6-Position durch Fluor, Chlor, Brom, Methyl, Trifluormethyl oder Trifluormethoxy, substituiert sein können, oder für 1,3-Thiazol-5-yl steht, das in 2-Position durch Chlor oder Methyl substituiert sein kann, oder für Pyrid-3-yl der folgenden Formel 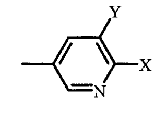 steht, in der
X für Halogen, C₁-C₁₂-Alkyl oder C₁-C₁₂-Halogenalkyl steht und
Y für Halogen, C₁-C₁₂-Alkyl, C₁-C₁₂-Halogenalkyl, C₁-C₁₂-Halogenalkoxy, Azido oder Cyan steht,
und in Formel (II)
E für eine Abgangsgruppe steht ausgewählt unter Chlor, Brom, Jod, eine aktivierte Hydroxyverbindung, Mesylat, Tosylat und SO₂CH₃,
und
Schritt (ii): Entfernung der Allylgruppe aus der in Schritt (i) erhaltenen Verbindung der Formel (III) wodurch ein Difluorethylamin-Derivat der Formel (IV) oder ein Salz davon erhalten wird, gegebenenfalls in Gegenwart eines Katalysators und gegebenenfalls in Gegenwart eines Nucleophils.

2. Das Verfahren nach Anspruch 1, wobei Schritt (ii) in Gegenwart eines Katalysators der ein oder mehrere Metalle der Gruppen 8 - 10 des Periodensystems enthält, und gegebenenfalls in Gegenwart eines Nucleophils durchgeführt wird, wobei das Nucleophil ausgewählt ist unter Hydroxiden, Alkoholaten, Thiolaten, Carbanionen, Halogenide, Peroxide, Cyanide und Azide, Thiole, Sulfinsäuren, 2-Mercaptobenzoesäure, Malonsäure und deren Derivate und β-Dicarbonylverbindungen, Barbitursäuren, N,N'-Dimethylbarbitursäure, Amine und Ethanolamin.

3. Das Verfahren nach Anspruch 2, wobei der Katalysator ein Palladium-Katalysator ist.

4. Das Verfahren nach Anspruch 3, wobei der Katalysator ausgewählt ist unter Palladium(0)-Katalysatoren, Palladium-10%ig auf Kohle, Palladium(II)chlorid, Palladium(II)acetat, Bis(acetylacetonat)palladium(II), Dichlorobis(triphenylphosphin)palladium(II), Tetrakis(triethylphosphin)palladium und Tetrakis(triphenylphosphin)palladium.

5. Das Verfahren nach einem der Ansprüche 1 bis 4, wobei die anorganische Base ausgewählt ist unter Hydriden, Hydroxiden, Amiden, Alkoholaten, Acetaten, Fluoriden, Phosphaten, Carbonaten und Hydrogencarbonaten von Alkali- oder Erdalkalimetallen, Natriumamid, Natriumhydrid, Lithiumdiisopropylamid, Natriummethanolat, Kalium-tert-butanolat, Natriumhydroxid, Kaliumhydroxid, Natriumacetat, Natriumphosphat, Kaliumphosphat, Kaliumfluorid, Caesiumfluorid, Natriumcarbonat, Kaliumcarbonat, Kaliumhydrogencarbonat, Natriumhydrogencarbonat und Caesiumcarbonat, und die organische Base ausgewählt ist unter Amine, substituierte oder unsubstituierte Pyridine und substituiertes oder unsubstituiertes Triethylamin, Trimethylamin, N,N-Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylethyldeniamin, N,N-Dimethyl-1,4-diazacyclohexan, N,N-Di-ethyl-1,4-diazacyclohexan, 1,8-Bis-(Dimethylamino)naphthalin, Diazabicyclooctan (DABCO), Diazabicyclononan (DBN), Diazabicycloundecan (DBU), Butylimidazol und Methylimidazol.

6. Das Verfahren nach einem der Ansprüche 1 bis 5 worin in Formel (II) E für Chlor, Brom oder Mesylat steht.

7. Das Verfahren nach einem der Ansprüche 1 bis 6, worin in den Formeln (II), (III), und (IV) A für 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 2-Chlor-1,3-thiazol-5-yl oder 2-Methyl-1,3-thiazol-5-yl, 5,6-Difluor-pyrid-3-yl, 5-Chlor-6-fluor-pyrid-3-yl, 5-Brom-6-fluor-pyrid-3-yl, 5-Fluor-6-chlor-pyrid-3-yl, 5,6-dichlor-pyrid-3-yl, 5-Brom-6-chlor-pyrid-3-yl, 5-Fluor-6-brom-pyrid-3-yl, 5-Chlor-6-brom-pyrid-3-yl, 5-Methyl-6-fluor-pyrid-3-yl, 5-Methyl-6-chlor-pyrid-3-yl, 5-Methyl-6-brom-pyrid-3-yl, 5-Difluormethyl-6-fluor-pyrid-3-yl oder 5-Difluormethyl-6-chlor-pyrid-3-yl steht.

8. Das Verfahren nach einem der Ansprüche 1 bis 6, worin in den Formeln (II), (III), und (IV) A für 6-Fluor-pyrid-3-yl, 6-Chlor-pyrid-3-yl, 6-Brompyrid-3-yl, 2-Chlor-1,3-thiazol-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl oder 5-Difluormethyl-6-chlor-pyrid-3-yl steht.

9. Das Verfahren nach einem der Ansprüche 1 bis 6, worin in den Formeln (II), (III), und (IV) A für 6-Chlor-pyrid-3-yl steht.

10. Das Verfahren nach einem der Ansprüche 1 bis 6, worin in den Formeln (II), (III), und (IV) A für 6-Brom-pyrid-3-yl steht.

11. Das Verfahren nach einem der Ansprüche 1 bis 6, worin in den Formeln (II), (III), und (IV) A für 2-Chlor-1,3-thiazol-5-yl steht.

12. Verfahren nach einem der Ansprüche 1 bis 6, worin in den Formeln (II), (III), und (IV) A für 5-Fluor-6-chlor-pyrid-3-yl steht.

13. Das Verfahren nach einem der Ansprüche 1 bis 6, worin in den Formeln (II), (III), und (IV) A für 5-Fluor-6-brom-pyrid-3-yl steht.

14. Eine Verbindung der Formel (III) 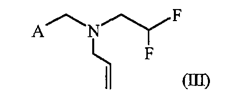 worin
A für Pyrid-2-yl, Pyrid-4-yl oder Pyrid-3-yl steht, die gegebenenfalls in 6-Position durch Fluor, Chlor, Brom, Methyl, Trifluormethyl oder Trifluormethoxy, substituiert sein können, oder für 1,3-Thiazol-5-yl steht, das in 2-Position durch Chlor oder Methyl substituiert sein kann, oder für Pyrid-3-yl der folgenden Formel 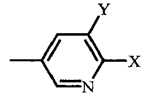 steht, in der
X für Halogen, C₁-C₁₂-Alkyl oder C₁-C₁₂-Halogenalkyl steht und
Y für Halogen, C₁-C₁₂-Alkyl, C₁-C₁₂-Halogenalkyl, C₁-C₁₂-Halogenalkoxy, Azido oder Cyan steht.

15. Die Verbindung der Formel (III) nach anspruch 14, worin A für 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 2-Chlor-1,3-thiazol-5-yl oder 2-Methyl-1,3-thiazol-5-yl, 5,6-Difluor-pyrid-3-yl, 5-Chlor-6-fluor-pyrid-3-yl, 5-Brom-6-fluor-pyrid-3-yl, 5-Fluor-6-chlor-pyrid-3-yl, 5,6-dichlor-pyrid-3-yl, 5-Brom-6-chlor-pyrid-3-yl, 5-Fluor-6-brom-pyrid-3-yl, 5-Chlor-6-brom-pyrid-3-yl, 5-Methyl-6-fluor-pyrid-3-yl, 5-Methyl-6-chlor-pyrid-3-yl, 5-Methyl-6-brom-pyrid-3-yl, 5-Difluormethyl-6-fluor-pyrid-3-yl oder 5-Difluormethyl-6-chlor-pyrid-3-yl steht.

16. Die Verbindung der Formel (III) nach Anspruch 14, worin A für 6-Chlor-pyrid-3-yl steht.

17. Die Verbindung der Formel (III) nach Anspruch 14, worin A für 6-Brom-pyrid-3-yl steht.

18. Die Verbindung der Formel (III) nach Anspruch 14, worin A für 2-Chlor-1,3-thiazol-5-yl steht.

19. Die Verbindung der Formel (III) nach Anspruch 14, worin A für 5-Fluor-6-chlor-pyrid-3-yl steht.

20. Die Verbindung der Formel (III) nach Anspruch 14, worin A für 5-Fluor-6-brom-pyrid-3-yl steht.

## Claims

1. A process for the preparation of 2,2-difluoroethylamine derivatives of the formula (IV) 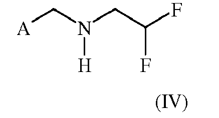 which comprises the following stages (i) and (ii):
stage (i): reaction of N-(2,2-difluoroethyl)prop-2-en-1-amine of the formula (I) 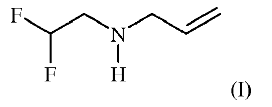 with a compound of the formula (II)
A-CH₂-E (II)
to give a compound of the formula (III) 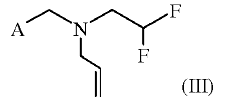 optionally in the presence of an inorganic or organic base, in which, in the formulae (II), (III) and (IV),
A is pyrid-2-yl, pyrid-4-yl or pyrid-3-yl, which can optionally be substituted in the 6-position by fluorine, chlorine, bromine, methyl, trifluoromethyl or trifluoromethoxy, or is 1,3-thiazol-5-yl, which can be substituted in the 2-position by chlorine or methyl, or is pyrid-3-yl of the following formula 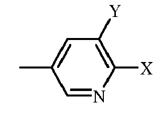 in which
X is halogen, C₁-C₁₂-alkyl or C₁-C₁₂-haloalkyl and
Y is halogen, C₁-C₁₂-alkyl, C₁-C₁₂-haloalkyl, C₁-C₁₂-haloalkoxy, azido or cyano,
and, in formula (II),
E is a leaving group chosen from chlorine, bromine, iodine, an activated hydroxyl compound, mesylate, tosylate and SO₂CH₃,
and
stage (ii): removal of the allyl group from the compound of the formula (III) obtained in stage (i), by which a difluoroethylamine derivative of the formula (IV) or a salt thereof is obtained, optionally in the presence of a catalyst and optionally in the presence of a nucleophile.

2. The process according to Claim 1, in which stage (ii) is carried out in the presence of a catalyst comprising one or more metals from Groups 8 - 10 of the Periodic Table and optionally in the presence of a nucleophile, the nucleophile being chosen from hydroxides, alkoxides, thiolates, carbanions, halides, peroxides, cyanides and azides, thiols, sulphinic acids, 2-mercaptobenzoic acid, malonic acid and derivatives thereof, and ß-dicarbonyl compounds, barbituric acids, N,N'-dimethylbarbituric acid, amines and ethanolamine.

3. The process according to Claim 2, in which the catalyst is a palladium catalyst.

4. The process according to Claim 3, in which the catalyst is chosen from palladium(0) catalysts, 10% palladium-on-charcoal, palladium(II) chloride, palladium(II) acetate, bis(acetylacetonate)palladium(II), dichlorobis(triphenylphosphine)palladium(II), tetrakis(triethylphosphine)palladium and tetrakis(triphenylphosphine)palladium.

5. The process according to one of Claims 1 to 4, in which the inorganic base is chosen from hydrides, hydroxides, amides, alkoxides, acetates, fluorides, phosphates, carbonates and hydrogencarbonates of alkali metals or alkaline earth metals, sodamide, sodium hydride, lithium diisopropylamide, sodium methoxide, potassium tert-butoxide, sodium hydroxide, potassium hydroxide, sodium acetate, sodium phosphate, potassium phosphate, potassium fluoride, caesium fluoride, sodium carbonate, potassium carbonate, potassium hydrogencarbonate, sodium hydrogencarbonate and caesium carbonate, and the organic base is chosen from amines, substituted or unsubstituted pyridines and substituted or unsubstituted triethylamine, trimethylamine, N,N-diisopropylethylamine, tri-n-propylamine, tri-n-butylamine, tri-n-hexylamine, tricyclohexylamine, N-methylcyclohexylamine, N-methylpyrrolidine, N-methylpiperidine, N-ethylpiperidine, N,N-dimethylaniline, N-methylmoipholine, pyridine, 2-, 3- or 4-picoline, 2-methyl-5-ethylpyridine, 2,6-lutidine, 2,4,6-collidine, 4-dimethylaminopyridine, quinoline, quinaldine,
N,N,N,N-tetramethylethylenediamine, N,N-dimethyl-1,4-diazacyclohexane, N,N-diethyl-1,4-diazacyclohexane, 1,8-bis(dimethylamino)naphthalene, diazabicyclooctane (DABCO), diazabicyclononane (DBN), diazabicycloundecane (DBU), butylimidazole and methylimidazole.

6. The process according to one of Claims 1 to 5, in which, in formula (II), E is chlorine, bromine or mesylate.

7. The process according to one of Claims 1 to 6, in which, in the formulae (II), (III) and (IV), A is 6-fluoropyrid-3-yl, 6-chloropyrid-3-yl, 6-bromopyrid-3-yl, 6-methylpyrid-3-yl, 6-(trifluoromethyl)pyrid-3-yl, 6-(trifluoromethoxy)pyrid-3-yl, 2-chloro-1,3-thiazol-5-yl or 2-methyl-1,3-thiazol-5-yl, 5,6-difluoropyrid-3-yl, 5-chloro-6-fluoropyrid-3-yl, 5-bromo-6-fluoropyrid-3-yl, 5-fluoro-6-chloropyrid-3-yl, 5,6-dichloropyrid-3-yl, 5-bromo-6-chloropyrid-3-yl, 5-fluoro-6-bromopyrid-3-yl, 5-chloro-6-bromopyrid-3-yl, 5-methyl-6-fluoropyrid-3-yl, 5-methyl-6-chloropyrid-3-yl, 5-methyl-6-bromopyrid-3-yl, 5-difluoromethyl-6-fluoropyrid-3-yl or 5-difluoromethyl-6-chloropyrid-3-yl.

8. The process according to one of Claims 1 to 6, in which, in the formulae (II), (III) and (IV), A is 6-fluoropyrid-3-yl, 6-chloropyrid-3-yl, 6-bromopyrid-3-yl, 2-chloro-1,3-thiazol-5-yl, 5-fluoro-6-chloropyrid-3-yl, 5,6-dichloropyrid-3-yl, 5-bromo-6-chloropyrid-3-yl, 5-fluoro-6-bromopyrid-3-yl, 5-chloro-6-bromopyrid-3-yl, 5,6-dibromopyrid-3-yl, 5-methyl-6-chloropyrid-3-yl or 5-difluoromethyl-6-chloropyrid-3-yl.

9. The process according to one of Claims 1 to 6, in which, in the formulae (II), (III) and (IV), A is 6-chloropyrid-3-yl.

10. The process according to one of Claims 1 to 6, in which, in the formulae (II), (III) and (IV), A is 6-bromopyrid-3-yl.

11. The process according to one of Claims 1 to 6, in which, in the formulae (II), (III) and (IV), A is 2-chloro-1,3-thiazol-5-yl.

12. Process according to one of Claims 1 to 6, in which, in the formulae (II), (III) and (IV), A is 5-fluoro-6-chloropyrid-3-yl.

13. The process according to one of Claims 1 to 6, in which, in the formulae (II), (III) and (IV), A is 5-fluoro-6-bromopyrid-3-yl.

14. A compound of the formula (III) 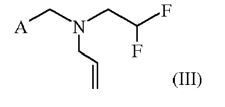 in which
A is pyrid-2-yl, pyrid-4-yl or pyrid-3-yl, which can optionally be substituted in the 6-position by fluorine, chlorine, bromine, methyl, trifluoromethyl or trifluoromethoxy, or is 1,3-thiazol-5-yl, which can be substituted in the 2-position by chlorine or methyl, or is pyrid-3-yl of the following formula 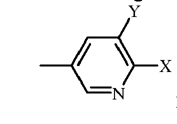 in which
X is halogen, C₁-C₁₂-alkyl or C₁-C₁₂-haloalkyl and
Y is halogen, C₁-C₁₂-alkyl, C₁-C₁₂-haloalkyl, C₁-C₁₂-haloalkoxy, azido or cyano.

15. The compound of the formula (III) according to Claim 14, in which A is 6-fluoropyrid-3-yl, 6-chloropyrid-3-yl, 6-bromopyrid-3-yl, 6-methylpyrid-3-yl, 6-(trifluoromethyl)pyrid-3-yl, 6-(trifluoromethoxy)pyrid-3-yl, 2-chloro-1,3-thiazol-5-yl or 2-methyl-1,3-thiazol-5-yl, 5,6-difluoropyrid-3-yl, 5-chloro-6-fluoropyrid-3-yl, 5-bromo-6-fluoropyrid-3-yl, 5-fluoro-6-chloropyrid-3-yl, 5,6-dichloropyrid-3-yl, 5-bromo-6-chloropyrid-3-yl, 5-fluoro-6-bromopyrid-3-yl, 5-chloro-6-bromopyrid-3-yl, 5-methyl-6-fluoropyrid-3-yl, 5-methyl-6-chloropyrid-3-yl, 5-methyl-6-bromopyrid-3-yl, 5-difluoromethyl-6-fluoropyrid-3-yl or 5-difluoromethyl-6-chloropyrid-3-yl.

16. The compound of the formula (III) according to Claim 14, in which A is 6-chloropyrid-3-yl.

17. The compound of the formula (III) according to Claim 14, in which A is 6-bromopyrid-3-yl.

18. The compound of the formula (III) according to Claim 14, in which A is 2-chloro-1,3-thiazol-5-yl.

19. The compound of the formula (III) according to Claim 14, in which A is 5-fluoro-6-chloropyrid-3-yl.

20. The compound of the formula (III) according to Claim 14, in which A is 5-fluoro-6-bromopyrid-3-yl.

## Revendications

1. Procédé pour la préparation de dérivés de 2,2-difluoroéthylamine de formule (IV) 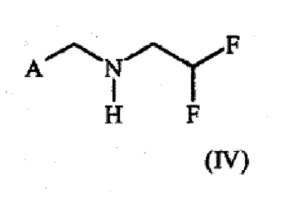 qui comprend les étapes (i) et (ii) suivantes :
étape (i) : mise en réaction de N-(2,2-difluoroéthyl)-prop-2-én-1-amine de formule (I) 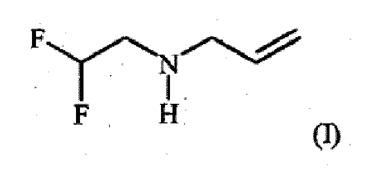 avec un composé de formule (II)
A-CH₂-E (II)
pour l'obtention d'un composé de formule (III) 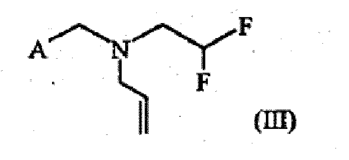 éventuellement en présence d'une base inorganique ou organique,
dans les formules (II), (III) et (IV)
A représentant un groupe pyrid-2-yle, pyrid-4-yle ou pyrid-3-yle, qui peuvent éventuellement être substitués en position 6 par fluoro, chloro, bromo, méthyle, trifluorométhyle ou trifluorométhoxy, ou représentant un groupe 1,3-thiazol-5-yle qui peut être substitué en position 2 par chloro ou méthyle, ou représentant un groupe pyrid-3-yle de formule suivante 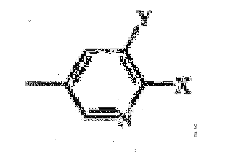 , dans laquelle
X représente un atome d'halogène, un groupe alkyle en C₁-C₁₂ ou halogénoalkyle(C₁-C₁₂) et
Y représente un atome d'halogène, un groupe alkyle en C₁-C₁₂, halogénoalkyle (C₁-C₁₂) , halogénoalcoxy(C₁-C₁₂), azido ou cyano,
et dans la formule (II)
E représentant un groupe partant choisi parmi chloro, bromo, iodo, un composé hydroxylé activé, mésylate, tosylate et SO₂CH₃,
et
étape (ii) : élimination du groupe allyle du composé de formule (III) obtenu dans l'étape (i), pour l'obtention d'un dérivé de difluoroéthylamine de formule (IV) ou d'un sel de celui-ci, éventuellement en présence d'un catalyseur et éventuellement en présence d'un composé nucléophile.

2. Procédé selon la revendication 1, dans lequel on effectue l'étape (ii) en présence d'un catalyseur qui contient un ou plusieurs métaux des groupes 8 - 10 du système périodique, et éventuellement en présence d'un composé nucléophile, le composé nucléophile étant choisi parmi des hydroxydes, alcoolates, thiolates, carbanions, halogénures, peroxydes cyanures et azides, thiols, acides sulfiniques, l'acide 2-mercaptobenzoïque, l'acide malonique et ses dérivés et des composés β-dicarbonyle, les acides barbituriques, l'acide N,N'-diméthylbarbiturique, des amines et l'éthanolamine.

3. Procédé selon la revendication 2, dans lequel le catalyseur est un catalyseur au palladium.

4. Procédé selon la revendication 3, dans lequel le catalyseur est choisi parmi des catalyseurs au palladium(0), le palladium à 10 % sur charbon, le chlorure de palladium(II), l'acétate de palladium(II), le bis(acétylacétonate)palladium(II), le dichlorobis-(triphénylphosphine)palladium(II), le tétrakis-(triéthylphosphine)palladium et le tétrakis(triphényl-phosphine)palladium.

5. Procédé selon l'une quelconque des revendications 1 à 4, dans lequel la base inorganique est choisie parmi des hydrures, hydroxydes, amides, alcoolates, acétates, fluorures, phosphates, carbonates et hydrogénocarbonates de métaux alcalins ou alcalino-terreux, l'amidure de sodium, l'hydrure de sodium, le diisopropylamidure de lithium, le méthanolate de sodium, le tert-butanolate de potassium, l'hydroxyde de sodium, l'hydroxyde de potassium, l'acétate de sodium, le phosphate de sodium, le phosphate de potassium, le fluorure de potassium, le fluorure de césium, le carbonate de sodium, le carbonate de potassium, l'hydrogénocarbonate de potassium, l'hydrogéno-carbonate de sodium et le carbonate de césium, et la base organique est choisie parmi des amines, des pyridines substituées ou non substituées et la triéthylamine substituée ou non substituée, la triméthylamine, la N,N-diisopropyléthylamine, la tri-n-propylamine, la tri-n-butylamine, la tri-n-hexylamine, la tricyclohexylamine, la N-méthyl-cyclohexylamine, la N-méthylpyrrolidine, la N-méthylpipéridine, la N-éthylpipéridine, la N,N-diméthylaniline, la N-méthylmorpholine, la pyridine, la 2-, 3-, 4-picoline, la 2-méthyl-5-éthyl-pyridine, la 2,6-lutidine, la 2,4,6-collidine, la 4-diméthylamino-pyridine, la quinoléine, la quinaldine, la N,N,N,N-tétraméthyléthylènediamine, le N,N-diméthyl-1,4-diazacyclohexane, le N,N-di-éthyl-1,4-diazacyclohexane, le 1,8-bis-(diméthylamino)naphtalène, le diazabicyclooctane (DABCO), le diazabicyclononane (DBN), le diazabicyclo-undécane (DBU), le butylimidazole et le méthylimidazole.

6. Procédé selon l'une quelconque des revendications 1 à 5, dans lequel dans la formule (II) E représente chloro, bromo ou mésylate.

7. Procédé selon l'une quelconque des revendications 1 à 6, dans lequel dans les formules (II), (III) et (IV) A représente le groupe 6-fluoro-pyrid-3-yle, 6-chloro-pyrid-3-yle, 6-bromo-pyrid-3-yle, 6-méthyl-pyrid-3-yle, 6-trifluorométhylpyrid-3-yle, 6-trifluorométhoxypyrid-3-yle, 2-chloro-1,3-thiazol-5-yle ou 2-méthyl-1,3-thiazol-5-yle, 5,6-difluoro-pyrid-3-yle, 5-chloro-6-fluoro-pyrid-3-yle, 5-bromo-6-fluoro-pyrid-3-yle, 5-fluoro-6-chloro-pyrid-3-yle, 5,6-dichloro-pyrid-3-yle, 5-bromo-6-chloro-pyrid-3-yle, 5-fluoro-6-bromo-pyrid-3-yle, 5-chloro-6-bromo-pyrid-3-yle, 5-méthyl-6-fluoro-pyrid-3-yle, 5-méthyl-6-chloro-pyrid-3-yle, 5-méthyl-6-bromo-pyrid-3-yle, 5-difluoro-méthyl-6-fluoro-pyrid-3-yle ou 5-difluorométhyl-6-chloro-pyrid-3-yle.

8. Procédé selon l'une quelconque des revendications 1 à 6, dans lequel dans les formules (II), (III) et (IV) A représente le groupe 6-fluoro-pyrid-3-yle, 6-chloro-pyrid-3-yle, 6-bromopyrid-3-yle, 2-chloro-1,3-thiazol-5-yle, 5-fluoro-6-chloro-pyrid-3-yle, 5,6-dichloro-pyrid-3-yle, 5-bromo-6-chloro-pyrid-3-yle, 5-fluoro-6-bromo-pyrid-3-yle, 5-chloro-6-bromo-pyrid-3-yle, 5,6-dibromo-pyrid-3-yle, 5-méthyl-6-chloro-pyrid-3-yl ou 5-difluorométhyl-6-chloro-pyrid-3-yle.

9. Procédé selon l'une quelconque des revendications 1 à 6, dans lequel dans les formules (II), (III) et (IV) A représente le groupe 6-chloro-pyrid-3-yle.

10. Procédé selon l'une quelconque des revendications 1 à 6, dans lequel dans les formules (II), (III) et (IV) A représente le groupe 6-bromo-pyrid-3-yle.

11. Procédé selon l'une quelconque des revendications 1 à 6, dans lequel dans les formules (II), (III) et (IV) A représente le groupe 2-chloro-1,3-thiazol-5-yle.

12. Procédé selon l'une quelconque des revendications 1 à 6, dans lequel dans les formules (II), (III) et (IV) A représente le groupe 5-fluoro-6-chloro-pyrid-3-yle.

13. Procédé selon l'une quelconque des revendications 1 à 6, dans lequel dans les formules (II), (III) et (IV) A représente le groupe 5-fluoro-6-bromo-pyrid-3-yle.

14. Composé de formule (III) 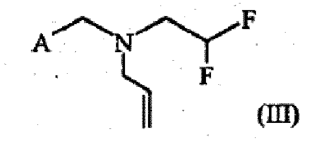 dans laquelle
A représente un groupe pyrid-2-yle, pyrid-4-yle ou pyrid-3-yle, qui peuvent éventuellement être substitués en position 6 par fluoro, chloro, bromo, méthyle, trifluorométhyle ou trifluorométhoxy, ou représente un groupe 1,3-thiazol-5-yle qui peut être substitué en position 2 par chloro ou méthyle, ou représente un groupe pyrid-3-yle de formule suivante 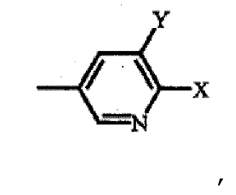 dans laquelle
X représente un atome d'halogène, un groupe alkyle en C₁-C₁₂ ou halogénoalkyle(C₁-C₁₂) et
Y représente un atome d'halogène, un groupe alkyle en C₁-C₁₂, halogénoalkyle (C₁-C₁₂), halogénoalcoxy(C₁-C₁₂), azido ou cyano.

15. Composé de formule (III) selon la revendication 14, dans lequel A représente le groupe 6-fluoro-pyrid-3-yle, 6-chloro-pyrid-3-yle, 6-bromo-pyrid-3-yle, 6-méthyl-pyrid-3-yle, 6-trifluorométhyl-pyrid-3-yle, 6-trifluorométhoxypyrid-3-yle, 2-chloro-1,3-thiazol-5-yle ou 2-méthyl-1,3-thiazol-5-yle, 5,6-difluoro-pyrid-3-yle, 5-chloro-6-fluoro-pyrid-3-yle, 5-bromo-6-fluoro-pyrid-3-yle, 5-fluoro-6-chloro-pyrid-3-yle, 5,6-dichloro-pyrid-3-yle, 5-bromo-6-chloro-pyrid-3-yle, 5-fluoro-6-bromo-pyrid-3-yle, 5-chloro-6-bromo-pyrid-3-yle, 5-méthyl-6-fluoro-pyrid-3-yle, 5-méthyl-6-chloro-pyrid-3-yle, 5-méthyl-6-bromo-pyrid-3-yle, 5-difluorométhyl-6-fluoro-pyrid-3-yle ou 5-difluorométhyl-6-chloro-pyrid-3-yle.

16. Composé de formule (III) selon la revendication 14, dans lequel A représente le groupe 6-chloro-pyrid-3-yle.

17. Composé de formule (III) selon la revendication 14, dans lequel A représente le groupe 6-bromo-pyrid-3-yle.

18. Composé de formule (III) selon la revendication 14, dans lequel A représente le groupe 2-chloro-1,3-thiazol-5-yle.

19. Composé de formule (III) selon la revendication 14, dans lequel A représente le groupe 5-fluoro-6-chloro-pyrid-3-yle.

20. Composé de formule (III) selon la revendication 14, dans lequel A représente le groupe 5-fluoro-6-bromo-pyrid-3-yle.
